# EUROPÄISCHE PATENTSCHRIFT

(11) **EP 1 151 082 B1**
(45) Veröffentlichungstag und Bekanntmachung des Hinweises auf die Patenterteilung: **08.03.2006**
(21) Anmeldenummer: 99963551.9
(22) Anmeldetag: 15.12.1999
(51) Int. Cl.: C12N 5/10, C12N 5/00, C12N 15/86, A61K 48/00, C12P 21/00, G01N 33/50, C12Q 1/02

(54) **SEBOZYTEN, SEBOZYTEN-ZELLINIE UND DEREN VERWENDUNGEN**
SEBOCYTES, SEBOCYTE CELL LINES AND APPLICATIONS THEREOF
SEBOCYTES, LIGNEES DE SEBOCYTES ET LEURS UTILISATIONS

(30) Priorität: 01.02.1999 DE 19903920
(43) Veröffentlichungstag der Anmeldung: 07.11.2001
(73) Patentinhaber: Zouboulis, Christos, 12200 Berlin (DE)
(72) Erfinder: Zouboulis, Christos, 12200 Berlin (DE)
(74) Vertreter: TBK-Patent
(86) Internationale Anmeldenummer: PCT/EP1999/009988
(87) Internationale Veröffentlichungsnummer: WO 2000/046353

(56) Entgegenhaltungen:
- WO-A-92/17569
- WO-A-98/08089
- WO-A-98/56897
- ZOUBOULIS C C ET AL: "The human sebocyte culture model provides new insights into development and management of seborrhoea and acne." DERMATOLOGY (BASEL) 1998, Bd. 196, Nr. 1, 1998, Seiten 21-31, XP000885771 ISSN: 1018-8665
- BRYAN TRACY M ET AL: "SV40-induced immortalization of human cells." CRITICAL REVIEWS IN ONCOGENESIS 1994, Bd. 5, Nr. 4, 1994, Seiten 331-357, XP000885786 ISSN: 0893-9675
- ZOUBOULIS CHRISTOS C ET AL: "Establishment and characterization of an immortalized human sebaceous gland cell line (SZ95)." JOURNAL OF INVESTIGATIVE DERMATOLOGY DEC., 1999, Bd. 113, Nr. 6, Dezember 1999 (1999-12), Seiten 1011-1020, XP000885609 ISSN: 0022-202X
- CHUAN-KUI J., ET AL.: "Comparison of methods for Transfection of Human Epidermal Keratinocytes" J. INVEST. DERMATOL., Bd. 97, 1991, Seiten 969-973, XP000999993
- THIBOUTOT D., ET AL.: "The Melanocortin 5 Receptor is Expressed in Human Sebaceous Glands and Rat Preputial Cells" J. INVEST. DERMATOL., Bd. 115, 2000, Seiten 614-619, XP000999971

## Beschreibung

Die vorliegende Erfindung betrifft fetthaltige und talgproduzierende Zellen der Haut und Schleimhaut, auch Sebozyten genannt. Insbesondere betrifft sie Talgdrüsenzellen und eine Talgdrüsenzellinie mit der Eigenschaft, über viele Subkulturen hinweg weitergezüchtet werden zu können. Die Sebozyten eignen sich hervorragend für nützliche Anwendungen, beispielsweise zur Untersuchung der Physiologie und Pathophysiologie der menschlichen oder der tierischen Talgdrüse, zur Untersuchung der Entstehung der Akne, der Seborrhoe bzw. anderer Erkrankungen, zum Testen der Wirkung verschiedener Substanzen und von Medikamenten, zur Entwicklung von Zellkultursystemen aus zweidimensionalen oder dreidimensionalen Zellansammlungen und Konstruktionen organähnlicher Strukturen, und zur Herstellung von Produkten, die von diesen Zellen stammen.

Zunehmende Anzeichen sprechen dafür, daß die Sebozyten eine kritische Rolle in pathophysiologischen Prozessen und in Krankheiten des Talgdrüsen-Haar-Apparates, insbesondere bei der Akne (Gollnick et al. J. Dermatol. 1991;18:489-499; Brown und Shalita. Lancet 1998;351:1871-1876; Cunliffe. Dermatology 1998;196:9-15; Strauss. Dermatology 1998;196:182-184) spielen. Ein Großteil unseres Verständnisses der Physiologie und Pathophysiologie der Talgdrüse stammen aus experimentellen Tiermodellen (Pochi, in Models in Dermatology, Vol. 2, N. Lowe and H. Maibach, editors, Basel, 1985;70-75). Es wurde aber gefunden, daß Tiermodelle keine vernünftigen Voraussagen zur Beurteilung der Wirkungen von Anti-Akne-Medikamenten beim Menschen zuließen (Geiger. Dermatology 1995;191:305-310). Die Tatsache, daß Akne lediglich beim Menschen auftritt und daß die sekretorische Aktivität der Talgdrüse stark spezienspezifisch ist (Nikkari. J. Invest. Dermatol. 1974;257-267), führte zu der Suche nach menschlichen Modellen. Anfängliche Studien zur Beseitigung dieser Nachteile erfolgten anhand von menschlichen Hautproben, die man entweder in-vitro inkubierte (Hsia et al. Proc. Soc. Exp. Biol. Med. 1970;135:285-291; Cooper et al. Br. J. Dermatol. 1976;94:165-172; Sharp et al. J. Endrocrinol. 1976;70:491-499) oder auf Nacktmäusen transplantierte (Petersen et al. J. Clin. Invest. 1984;74:1358-1365). Grundlegende Studien über die Aktivität menschlicher Sebozyten sowie deren Regulierung waren jedoch erst in dem vergangenen Jahrzehnt möglich, als lebensfähige menschliche Talgdrüsen isoliert wurden (Kealey et al. Br. J. Dermatol. 1986;114:181-188) und das Kulturmodell menschlicher Sebozyten in vitro etabliert werden konnte (Xia et al. J. Invest. Dermatol. 1989;93:315-321).

Im Laufe der vergangenen Jahre wurden durch Modifikationen der Kulturtechnik von Xia et al. (1989) Verbesserungen hinsichtlich der Reproduzierbarkeit der Kultivierung menschlicher Sebozyten in vitro erzielt. So verzichteten Zouboulis et al. (Skin. Pharmacol. 1991;4:74-83) durch die Zugabe menschlichen Serums auf Hydrokortison im Kulturmedium. Lee (in Epithelia: Advances in Cell Physiology and Cell Culture; C.J. Jones, editors: Kluwer, Dordrecht, 1990;333-350) behandelte Talgdrüsen mit Kollagenase, bevor sie sie in serumfreiem Medium, welches mit Zusatzstoffen angereichert war, kultivierte. Ebenso konnten primäre Sebozytenkulturen erhalten werden, indem die 3T3-Fibroblastenschicht, die als Haftungsboden dient, weggelassen wurde (Akamatsu et al. J. Invest. Dermatol. 1992;99:509-511). Sekundäre Kulturen konnten in einem Medium, welches durch entfettetes Serum ergänzt wurde (Zouboulis et al. J. Invest. Dermatol. 1993;101:628-633), sowie in serumfreiem Keratinozyten-Basalmedium ohne Zusätze (Akamatsu et al. J. Invest. Dermatol. 1992;99:509-511) aufrechterhalten werden. Ferner wurde gezeigt, daß der Keratinozyten-Wachstumsfaktor (KGF) die Ausbeute und die Proliferation menschlicher Sebozyten merklich verbesserte (Chen et al. J. Invest. Dermatol. 1998;110:84-89).

Trotz dieser technischen Verbesserungen wird die weitere Entwicklung stark dadurch behindert, daß das Kultivieren einer großen Zahl von Sebozyten aus isolierten menschlichen Talgdrüsen schwierig ist. Insbesondere besteht die Schwierigkeit, das Zellenmaterial für eine lange Zeitdauer in Kultur zu halten. Als Grund dafür wird die Neigung der Sebozyten angenommen, sich zu differenzieren und durch eine spontane Zellmembranruptur und Freisetzung ihres Inhaltes zu sterben. Das beste Resultat wurde bisher von Fujie et al. (Arch. Dermatol. Res. 1996;288:703-708) erreicht, die Talgdrüsen auf der Grundlage der Technik von Xia et al. (1989) isolierten und Sebozyten mittels der Methode der dispergierten Zellkultur sechs Subkulturen in serumfreiem, Keratinozyten-Wachstumsmedium ohne Haftungszellschicht kultivierten.

Zouboulis, Ch. C. et al. Dermatology 1998, 196: 21-31, gibt einen Überblick über Erfahrungen, die im Zusammenhang mit der Kultivierung von Primär Kulturen menschliches Sebozyten gewonnen wurden.

WO 98/08089 offenbart Konditionell immortalisierte Zellen der Präputialdrüse der Maus.

Bryan, T.M. und Reddel R.R., Crit. Rev. in Oncogen., 5(4): 331-357 (1994) gibt einen Überblick über die SV40 induzierte Immortalisierung menschliches Zellen.

Es ist Aufgabe der vorliegenden Erfindung, menschliche Sebozyten bereitzustellen, die über eine höhere Anzahl von Subkulturen hinweg in Kultur gehalten werden können. Dabei soll sich die bereitgestellten Sebozyten in ihrem Erscheinungsbild den morphologischen, phänotypischen und funktionellen Charakteristika von lebenden, normalen, menschlichen Sebozyten ähneln bzw. soweit annähern, daß sie sich als zelluläres bzw. Zellkulturmodell für fetthaltige, talgproduzierende Zellen und insbesondere für Sebozyten zu physiologischen, pathophysiologischen und pharmazeutischen Untersuchungen gut eignen.

Die Erfindung wird durch die Bereitstellung von Sebozyten gelöst, die gemäß Anspruch 1 definiert sind. Sebozyten dieser Art liegen vor in der menschlichen Talgdrüsenzellinie SZ95, die bei der Deutschen Sammlung von Mikroorganismen und Zellkulturen GmbH (DSMZ) unter der Hinterlegungsnummer DSM ACC2383 hinterlegt wurde.

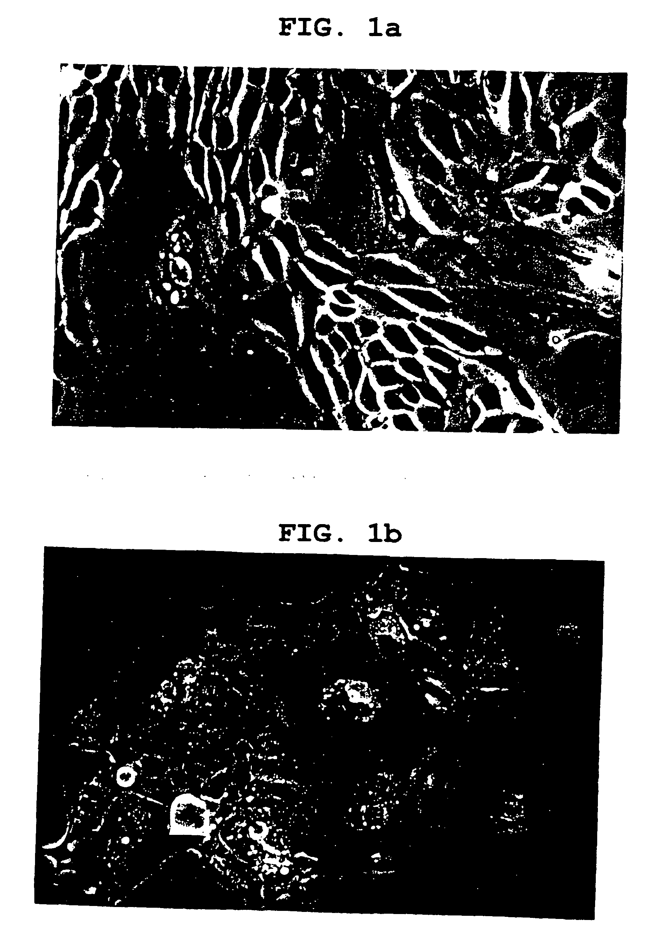

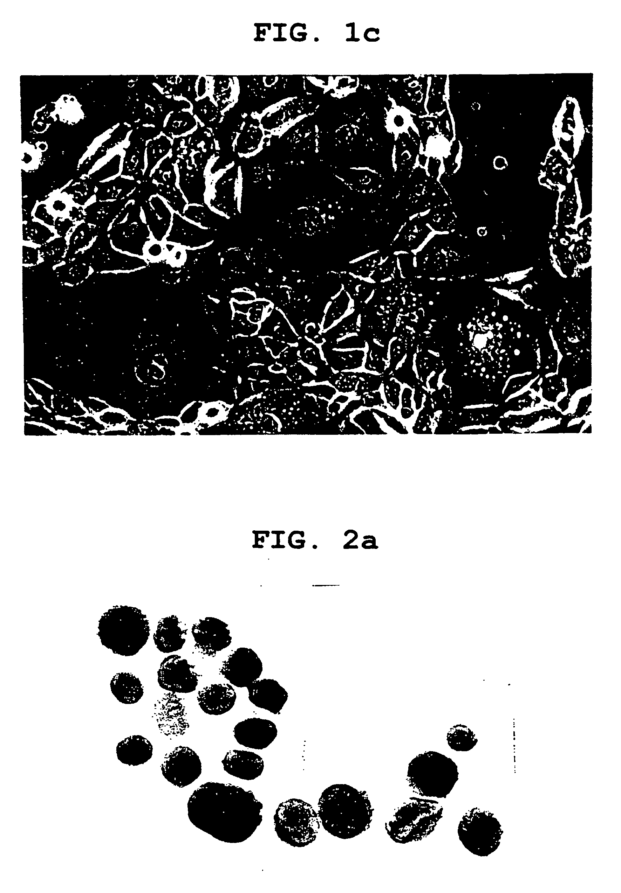

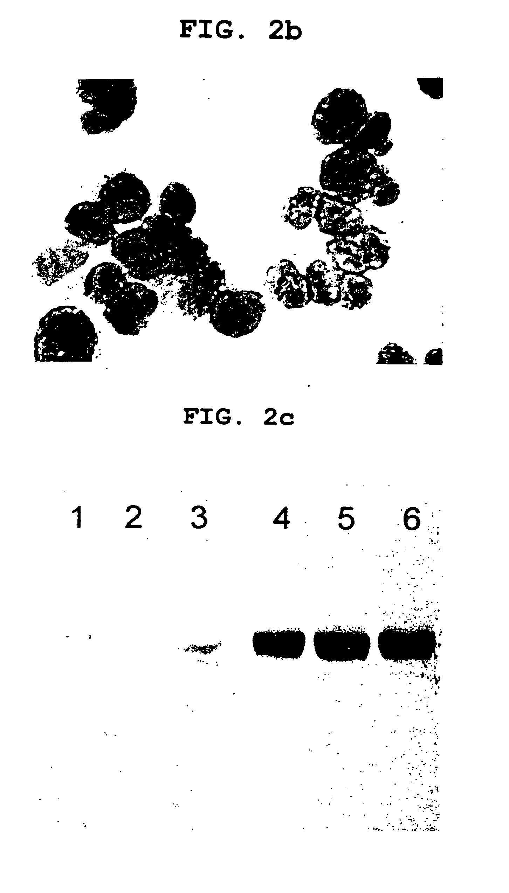

Die vorliegende Erfindung wird nachfolgend unter Bezugnahme auf die Abbildungen (Fig.) näher erläutert. Fig. 1 und Fig. 2 zeigen, daß die bereitgestellten immortalisierten Sebozyten SZ95 das epitheliale, polymorphe Aussehen der primären normalen Sebozyten, aus denen sie stammen (hier vom Menschen), behalten haben. Darüberhinaus exprimieren die bereitgestellten immortalisierten Sebozyten und ihre Klone (Klon=Zellen, die gesichert aus einer einzigen Zelle stammen) das charakteristische, 94-kD-große SV-40-Große-T-Antigen, mit dessen kodierenden DNA-Sequenz sie transfiziert wurden, auch in späteren Subkulturen. Die Fig. 1 zeigt (a) normale, menschliche Sebozyten der zweiten Subkultur, aus denen die bereitgestellten immortalisierten Sebozyten stammen, (b) eine adhärente Sebozytenkultur aus bereitgestellten immortalisierten Sebozyten in der ersten Subkultur, (c) bereitgestellte immortalisierte Sebozyten (50. Subkultur eines Klons). Alle Zellen zeigen eine ähnelnde epitheliale, polymorphe Struktur. Die Fig. 2. zeigt (a) Zytozentrifugen-Präparate bereitgestellter immortalisierter Sebozyten und (b) der endothelialen Zellkultur HMEC-1, die als positive Kontrolle diente, die mit einem monoklonalen Antikörper gegen das menschliche SV-40-Große-T-Antigen gefärbt sind. Beide Präparate sind positiv gefärbt und zeigen, daß das menschliche SV-40-Große-T-Antigen überwiegend im Zellkern, teils auch im Zellzytoplasma, lokalisiert ist. In (c) wird die Expression des menschlichen SV-40-Großen-T-Antigen in den bereitgestellten immortalisierten Sebozyten mittels Westernblot dargestellt. Während das menschliche SV-40-Große-T-Antigen bei nicht-transfizierten, normalen menschlichen Sebozyten (Spur 1) und normalen menschlichen epidermalen Keratinozyten (Spur 2) nicht nachweisbar war, wurde das charakteristische 94-kD-große Protein in Proteinextrakten der bereitgestellten immortalisierten Sebozyten in der 34. Subkultur (Spur 3) sowie von drei isolierten Klonen (Spur 4, 5 bzw. 6) nachgewiesen.

Die immortalisierten Sebozyten der vorliegenden Erfindung sind menschlichen Ursprungs. Die Bedeutung des Ausdrucks "Sebozyten" ist im weitesten Sinne zu verstehen, d.h. betreffend alle Zellen, die mehr oder weniger fetthaltig, talgproduzierend sind. Talg (Sebum) setzt sich überwiegend aus verschiedenen Fetten zusammen. Dabei kann der Fettinhalt der Zellen sowohl hinsichtlich der Fettfraktionen als auch hinsichtlich der Gehalte der Fettfraktionen variieren. In der Regel, aber nicht notwendigerweise, umfaßt der Fettinhalt der Zellen freie Fettsäuren, Triglyceride, Wachse, Squalen, freies Cholesterin, Cholesterinester, Dihydroxycholesterin und andere Steroide sowie andere Kohlenwasserstoffe. Insbesondere werden solche immortalisierten Sebozyten bevorzugt, die von der menschlichen Talgdrüsenzelle stammen. Eine besonders gute Eignung für medizinische Zwecke ergibt sich, wenn die Sebozyten aus menschlichen Gesichtstalgdrüsenzellen stammen.

Ein wesentliches Charakteristikum der erfindungsgemäßen Sebozyten besteht in ihrer Immortalisierung. Immortalisiert im Sinne der vorliegenden Erfindung bedeutet das grundsätzliche Aufrechterhalten des Vitalzustandes der Zellen über eine Vielzahl von Subkulturen hinweg. Die erfindungsgemäßen, immortalisierten Sebozyten SZ95 konnten in der zurückliegenden Beobachtungszeit von etwa 4½ Jahren über mehr als 50 Subkulturen in Kultur gehalten werden, wohingegen normale menschliche Sebozyten nur über drei bis sechs Subkulturen wachsen können, bevor sie absterben.

Die immortalisierten Sebozyten gemäß der vorliegenden Erfindung können erhalten werden, indem normale Sebozyten mit einer DNA transfiziert werden, die DNA-Sequenzen umfaßt, welche für das Große-T-Protein von SV-40 kodieren. Die immortalisierende Wirkung des SV-40-Großen-T-Antigens (Proteins) sowie die entsprechende Verwendung der kodierenden DNA-Sequenz zur Transfektion menschlicher Zellen ist an sich bekannt. So wurden immortalisierte Zellinien durch Transfektion mit einer für SV-40 T kodierenden DNA beispielsweise aus anderen Zellen epithelialen Ursprungs (siehe Tohyama et al. Tohoku. J. Exp. Med. 1997;182:75-82; Bae et al. Prostate 1998;34:275-282) sowie endothelialen Ursprungs (siehe Ades et al. J. Invest. Dermatol. 1992;99:683-690 und WO-A-92/17569) erhalten.

Es hat sich herausgestellt, daß eine Sebozyten-Transfektion nach einer Gentransfermethode mittels Anwendung kationischer Lipide (LIPOFECTIN-Reagens; das eine 1:1 (w/w) liposomale Formulierung der kationischen Lipide DOTMA [1,2-Diolyloxypropyl-3-trimethylammoniumchlorid] und DOPE [Diolylphosphatidylethanolamin] in membrangefiltertem Wasser [1 mg/ml] beinhaltet), bei der die Fremd-DNA über kationische Lipide-DNA-Komplexe durch Endozytose in die Zellen aufgenommen wird (siehe Wang et al. In. Vitro. Cell. Dev. Biol. 1991;27A:63-74; Staedel et al. J. Invest. Dermatol. 1994;102:768-772), gute Ergebnisse bei der Immortalisierung lieferte, wobei in der Transfektionsmischung vorzugsweise 0,25 bis 2,0 Vol.-% und insbesondere 2,0 Vol.-% LIPOFECTIN-Reagens sowie 0,05 bis 0,5 Gew.-% und insbesondere 0,5 Gew.-% Fremd-DNA in einem geeigneten Transfektionspuffer eingesetzt waren. Die Fremd-DNA, wie die für das SV-40-Große-T-Protein kodierende DNA, ist dabei üblicherweise in einem geeigneten Vektor insertiert, bei dem die Expression des SV-40-Großen-T-Proteins vorzugsweise mittels Promotor- und Enhancer-Sequenzen gesteigert ist. Sind die normalen menschlichen Sebozyten in der bevorzugten Ausführung durch die DNA transfiziert, die für das SV-40-Groß-T-Antigen kodiert, so ist zu erwarten, daß die bereitgestellten Sebozyten nach der erfolgreichen Transfektion und Immortalisierung das Große-T-Antigen von SV-40 exprimieren. Dies wurde für die bereitgestellten immortalisierten Sebozyten gemäß der vorliegenden Erfindung immunzytochemisch und durch Westernblot-Analysen unter Verwendung monoklonaler Antikörper gegen das SV-40-Groß-T-Antigen bestätigt.

Die so erhaltenen, immortalisierten Sebozyten liegen vorzugsweise in Form einer Zellinie vor und können in dieser Form in hervorragender Weise für die Anwendungszwecke eingesetzt werden.

Die immortalisierten Sebozyten gemäß der vorliegenden Erfindung wuchsen nach Anpassung an serumfreies Kulturmedium besser als nicht-transfizierte, normale menschliche Sebozyten, und sie hielten ihre Fähigkeit aufrecht, Talgdrüsen-spezifische Lipide zu synthetisieren - im Gegensatz zu den in serumfreiem Medium gehaltenen, nicht-transfizierten normalen menschlichen Sebozyten. Die erfindungsgemäßen immortalisierten Sebozyten können somit als stetig erneuerbare und vermehrungsfähige Zellinie dienen und in definierten Kulturmedien wachsen.

Ein besonderer Wert der erfindungsgemäßen, immortalisierten Sebozyten besteht darin, daß sie in morphologischer, phänotypischer und funktioneller Hinsicht Merkmale von nicht-transfizierten, normalen und differenzierenden Sebozyten aufweisen. Dadurch bieten sich die erfindungsgemäßen, immortalisierten Sebozyten in hervorragender Weise als Modell zu physiologischen, pathophysiologischen und pharmakologischen Untersuchungen an. Gleichzeitig wird der Nachteil der begrenzten Lebensfähigkeit herkömmlicher, in Kultur gehaltener normalen Sebozyten menschlichen Ursprungs vermieden. So konnte bestätigt werden, daß immortalisierte Sebozyten gemäß der vorliegenden Erfindung einen normalen Sebozyten-Phänotyp weitgehend aufrechterhalten und sich in funktioneller Hinsicht ähnlich wie nicht-transfizierte, normale menschliche Gesichtssebozyten verhalten können.

Es wurde festgestellt, daß die erfindungsgemäßen, immortalisierten Sebozyten bzw. die Sebozytenlinie (Sebozyten-Zellinie) eine polymorphe, epitheliale Erscheinung zeigen, die derjenigen nicht-transfizierter, normaler menschlicher Sebozyten ähnelt. In der Zellkultur erschienen Zellen unterschiedlicher Größe und intrazellulärer Struktur, was auf unterschiedliche Stadien in der Zellreifung hinwies. So wurden Zellen unterschiedlicher Größe, durchschnittlich bis hin zur 5-fachen bzw. 6-fachen Größe bei konfluentem Wachstum, beobachtet, was im wesentlichen der Zellgrößensteigerung von nicht-transfizierten, normalen menschlichen Sebozyten mit progressiver Differenzierung in vitro (durchschnittlich 4- bis 5,5-facher Größenunterschiede) entspricht. Des weiteren wurden wie bei nicht-transfizierten, normalen menschlichen Sebozyten reichlich Fettpartikel im Zytoplasma der erfindungsgemäßen, immortalisierten Sebozyten gefunden. Die Synthese der charakteristischen Talgdrüsenlipide Squalen und Wachsester, wie es für normale menschliche Sebozyten üblich ist, wurde im Rahmen der vorliegenden Erfindung experimentell bestätigt. Ferner synthetisierten die immortalisierten Sebozyten der vorliegenden Erfindung freie Fettsäuren - was wiederum mit den Erkenntnissen über nicht-transfizierten, normalen menschlichen Sebozyten in vitro korreliert - und zwar sogar nach einer bereits hohen Zahl von Subkulturen.

Auch Expressionsmarker, die einen Sebozytenursprung bestätigen und eine intakte Differenzierung aufzeigen, wurden als typische Anzeichen für Sebozyten bei der erfindungsgemäßen, immortalisierten Sebozyten bzw. Sebozytenlinie bestätigt. So exprimierten die erfindungsgemäßen, immortalisierten Sebozyten bzw. die Sebozytenlinie für Sebozyten typische Antigene der humanen polymorphen epithelialen Mukoproteingruppe, wie das Talgdrüsenantigen, humanes Milchfettglobulin-1 und -2, humanes epitheliales Sialomucin, das Thomsen-Friedenreich-Antigen, Muzin-ähnliches-Karzinom assoziiertes Antigen sowie epitheliales Membranantigen. Dies wurde im Rahmen der vorliegenden Erfindung immunozytochemisch und durch Westernblot-Analyse bestätigt. Darüber hinaus exprimierten die erfindungsgemäßen, immortalisierten Sebozyten bzw. die Sebozytenlinie die für nicht-transfizierte, normale menschliche Sebozyten typischen Keratin-Antigene, wie solche der Sub-Klassen 7, 13 und 19. Der Antigenphänotyp belegte somit den Sebozytenursprung sowie die Sebozytendifferenzierung.

Auch in funktioneller Hinsicht verhalten sich die immortalisierten Sebozyten bzw. die Sebozytenlinie der vorliegenden Erfindung ähnlich zu nicht-transfizierten, normalen menschlichen Sebozyten. So sprachen die immortalisierten Sebozyten der vorliegenden Erfindung auf die Einwirkung durch Androgene an, wie z.B. bei 5α-Dihydrotestosteron, indem ihre In-Vitro-Proliferation gesteigert wurde. Ferner besaßen die erfindungsgemäßen, immortalisierten Sebozyten bzw. die Sebozytenlinie die Fähigkeit, ihre Proliferation durch Einwirkung von Retinoiden, insbesondere solche des nichtaromatischen Typs (z.B. 13-cis-Retinsäure, all-trans-Retinsäure), zu verändern.

Eine bevorzugte Ausgestaltung der vorliegenden Erfindung besteht darin, daß die immortalisierten und vorzugsweise menschlichen Sebozyten kloniert sind. Dies hat den Vorteil, daß die immortalisierten Sebozyten bzw. die daraus entstehende Sebozytenlinie mittels der einheitlichen genomischen Basis gut definiert und spezifisch charakterisiert sind. Eine klonierte und immortalisierte menschliche Sebozytenlinie wurde geeigneterweise erhalten, indem immortalisierte Sebozyten in Kulturgefäßen solange verdünnt wurden, bis die Zellteilung von lediglich einer Zelle pro Kulturgefäß neu einsetzte. Dies konnte mittels mikroskopischer Beobachtungen überwacht und kontrolliert werden.

Somit wurde im Rahmen der vorliegenden Erfindung festgestellt, daß die erhaltenen, immortalisierten menschlichen Sebozyten bzw. die daraus entstehende Sebozytenlinie ihre Sebozytenidentität im Vergleich zu nicht-transfizierten, normalen menschlichen Sebozyten erhielten. Dies wurde durch Charakterisierungskontrollen und funktionelle Tests bestätigt.

Eine Sebozytenlinie mit der Bezeichnung SZ95, die die oben genannten Vorteile der vorliegenden Erfindung in sich vereinigt, stellt die unter der Hinterlegungsnummer ACC2383 bei der DSMZ hinterlegten Talgdrüsenzell (Sebozyten)-Linie dar.

Somit bieten die immortalisierten Sebozyten bzw. die Sebozytenlinie gemäß der vorliegenden Erfindung ausgezeichete Möglichkeiten für sehr nützliche Anwendungen. Generell können die Sebozyten bzw. die Sebozyten-Zellinie gemäß der Erfindung für diagnostische, für therapeutische oder für kosmetische Ansätze eingesetzt werden. Speziell dienen die oben beschriebenen Sebozyten bzw. die Sebozytenlinie zu Untersuchungen der Physiologie oder der Pathophysiologie von lipidhaltigen, talgproduzierenden Zellen, insbesondere von menschlichen oder tierischen Talgdrüsenzellen, sowie deren Rolle in pathophysiologischen Prozessen der Haut und in Hautkrankheiten wie z.B. der Akne. Mit Hilfe der Erfindung kann so die Entstehung der Akne und/oder der Seborrhoe und/oder anderer Erkrankungen, insbesondere von Hautkrankheiten, bei denen die Talgdrüsenfunktion eine Rolle spielt oder spielen kann, untersucht werden. Die Gegenstände der vorliegenden Erfindung dienen ferner als ausgezeichnete Modelle zum Testen und zur Beurteilung von Anti-Akne- und/oder Anti-Seborrhoe-Verbindungen oder -Mitteln aber auch von Mitteln gegen Erkrankungen, insbesondere von Hautkrankheiten, bei denen die Talgdrüsenfunktion eine Rolle spielt oder spielen kann. Gerade vor der Durchführung klinischer Studien sind solche In-Vitro-Untersuchungen zu pharmakologischen Eigenschaften von Medikamente nützlich.

Die oben beschriebenen Sebozyten oder Sebozytenlinie gemäß der Erfindung haben ferner den Vorteil, daß damit weitere Zellkultursysteme etabliert werden können. Dies schließt die Entwicklung von einfachen und von komplexen Zellkultursystemen ein. Einfache Zellkultursysteme bedeutet in der Regel zweidimensionale Einschicht- oder Mehrschicht-adhärente Kulturen oder nicht-adhärente Kulturen und werden beispielsweise gebildet, indem man die oben beschriebenen Sebozyten mit anderen Zellarten einmischt oder getrennt durch semi- oder nichtpermeablen Membranen kultiviert (Schwartz et al. J. Surg. Res. 1998;76:79-85; Nackman et al. Surgery. 1998;124:353-61). Komplexe Zellkultursysteme bedeutet in der Regel eine dreidimensionale Kultivierung einschichtiger oder mehrschichtiger Kulturen und werden beispielsweise gebildet, indem man die Zellen als Sphäroide, auf Sphäroide, in Kollagen oder in anderen Gelpräparaten oder in einer künstlichen Hautähnlichen Struktur kultiviert (Korff und Augustin. J. Cell. Biol. 1998;143:1341-1352; Hamamoto et al. J. Biochem. (Tokyo) 1998;124:972-979; Desoize et al. Anticancer. Res. 1998;18:4147-4158; Hamilton. Cancer. Lett. 1998;131:29-34; Niemann et al. J. Cell. Biol. 1998;143:533-545; Awata et al. J. Gastroenterol. Hepatol. 1998;13 Suppl:S55-61; Voura et al. Microsc. Res. Tech. 1998;43:265-275; Pipili-Synetos et al. Br. J. Pharmacol. 1998;125:1252-1257; Vasile et al. J. Histochem. Cytochem. 1999;47:159-168; Michalopoulos et al. Hepatology. 1999;29:90-100; Trent und Kirsner. Int. J. Clin. Pract. 1998;52:408-413; Fransson et al. Br. J. Dermatol. 1998;139:598-604; Konstantinova et al. Arch. Dermatol. Res. 1998;290:610-614; Black et al. FASEB. J. 1998;12:1331-1340; Zhao et al. Biochem. Biophys. Res. Commun. 1999;254:49-53).

Eine besonders brauchbare Nutzung betrifft die Bildung von dreidimensionalen Zellansammlungen oder die Konstuktionen bzw. Rekonstruktionen organähnlicher Strukturen aus den erfindungsgemäßen Sebozyten bzw. der erfindungsgemäßen Sebozytenlinie. Hierfür werden die Sebozyten allein, vorzugsweise aber zusammen mit weiteren, hautaufbauenden Zellen, insbesondere mit Keratinozyten, Fibroblasten, Melanozyten, Endothelzellen, Langerhans-Zellen und/oder Zellen aus dem Haarfollikel eingesetzt. Zur Bildung solcher dreidimensionaler Zellansammlungen oder Konstuktionen bzw. Rekonstruktionen organähnlicher Strukturen wird geeigneterweise zunächst ein Stützgerüst mit Kollagen oder anderen Gelpräparaten und/oder mit Stücken von inaktiviertem Gewebe bereitgestellt, und danach die genannten Zellen in oder auf dieses Stützgerüst ein- bzw. aufgebracht werden. Diese Methode ist dem Fachmann an sich bekannt und erste Präparate sind im Handel erhältlich (Trent und Kirsner. Int. J. Clin. Pract. 1998;52:408-413; Fransson et al. Br. J. Dermatol. 1998;139:598-604; Konstantinova et al. Arch. Dermatol. Res. 1998;290:610-614; Black et al. FASEB. J. 1998;12:1331-1340; Zhao et al. Biochem. Biophys. Res. Commun. 1999;254:49-53). Auf diese Weise wird eine "künstliche Haut" oder ein Hautersatz hergestellt, was ausgezeichnete Möglichkeiten für die Transplantationsmedizin, für die Rekonstruktion von defekten Hautpartien wie z.B. verbrannter Haut, oder zur Therapie von Hautläsionen bietet. Mit Hilfe der Erfindung kann diese "künstliche Haut" auch Fett/Talg in ausreichender Menge synthetisieren, wenn die erfindunggemäßen Sebozyten in diesem Konstrukt eingebracht werden.

Ein weiteres Gebiet sehr nützlicher Anwendungen betrifft die Herstellung von Produkten, die von den erfindungsgemäßen Sebozyten bzw. der erfindungsgemäßen Sebozytenlinie stammen. Dies schließt die Isolierung und Reinigung von zellulären Substanzen, wie Lipide, Proteine, DNA und/oder RNA, ein. Da die Zellen immortalisiert sind, stehen sie als stete Quelle für solche zelluläre Substanzen zur Verfügung. Spezielle Beispiele für sehr brauchbare Substanzen, die auf diese Weise aus den Zellen erhältlich sind, schließen ein: Hautlipide für die Anwendung in topischen Mitteln und Medikamenten, die im untenstehenden Beispiel 3 im Zusammenhang mit der phänotypischen Charakterisierung der Sebozyten genannten, antigenen Proteine, und ferner die Erzeugung von Plasmid-DNA oder Vektor-DNA. Die Erzeugung von Plasmid-DNA oder Vektor-DNA erfolgt auf dem Fachmann bekanntem gentechnologischem Wege; damit können z.B. Gene erfaßt werden, die die Lipidproduktion induzieren. Gerade mit solchen, geeigneten Plasmid- und Vektor-Konstrukten, wobei die Bildung viraler Vektoren ebenfalls in Betracht gezogen werden können, können wiederum andere Zellen oder Organismen modifiziert oder transfiziert werden.

Die vorliegende Erfindung wird nachstehend anhand nicht einschränkender Beispiele näher erläutert.

### Beispiele

Für die anschließend beschriebenen Beispiele können die nachfolgend beschriebenen materiellen und methodischen Ausgestaltungen Anwendung finden. Die Beispiele sind jedoch nicht einschränkend zu verstehen.

### Zellkulturen

Wenn nicht anders angegeben wurden alle Zellen als adhärente Kulturen in einem Medium gehalten, welches aus modifiziertem DMEM/Ham's F12-Medium (1:1)(erhältlich von Biochrom, Berlin, Deutschland) mit 2 mM N-Acetyl-L-Alanyl-L-Glutamin bestand, welches mit 10% hitzeinaktiviertem, fötalem Kälberserum (FCS; Biochrom) sowie 50 µg/ml Gentamycin (erhältlich von Gibco-BRL, Karlsruhe, Deutschland) ergänzt war. Die Kultur wurde bei 37°C in feuchter Atmosphäre, die 5% CO₂ enthielt, gehalten, und das Kulturmedium wurde alle 2 bis 3 Tage erneuert.

### Isolierung und Kultivierung normaler menschlicher Sebozyten

Normale Sebozyten wurden von der Gesichtshaut einer 87-jährigen weiblichen Patientin, die einer Operation unterzogen wurde, isoliert, wie es von Xia et al. (J. Invest. Dermatol. 1989;93:315-321) beschrieben wurde. Die isolierten Talgdrüsen wurden ohne Haftungszellschicht in dem Standardmedium kultiviert, welches mit 9 ng/ml Epidermal-Growth-Factor (EGF), 9 ng/ml Keratinocyte-Growth-Factor (KGF) (beides erhältlich von Boehringer Mannheim, Deutschland), 0,4 µg/ml Hydrocortison (erhältlich von Sigma, Deisenhofen, Deutschland) sowie 10⁻⁹ M Choleratoxin (erhältlich von Calbiochem, Bad Soden, Deutschland) ergänzt war. Kulturen primärer normaler Sebozyten ergaben sich als Auswuchs aus der Peripherie der Talgdrüsenläppchen.

### Immunzytochemische Untersuchungen

Dispergierte Zellen aus subkonfluente normale Sebozytenkulturen wurden auf Glas-Objektträgern mittels Zytozentrifugation angeheftet. Die Proben wurden luftgetrocknet und mittels kaltem Aceton über 10 Minuten fixiert. Die Präparate wurden dann mit dem entsprechenden monoklonalen Antikörper oder einem Kontrollantikörper für 30 Minuten bei Raumtemperatur inkubiert. Gebundene Antikörper wurden nachgewiesen durch Kopplung mit einer 1:100-Verdünnung eines monoklonalen Antikörperkonjugats aus Kaninchen-/Anti-Maus-IgG (H+L) und einem Alkalische Phosphatase/Anti-Alkalische Phosphatase-Komplex (erhältlich von Dianova, Hamburg, Deutschland) für 30 Minuten bei Raumtemperatur. Primäre und sekundäre monoklonale Antikörper wurden verdünnt in Lösungen, die 10% RPMI-1640 und 10% FCS bei einem pH von 7,4 enthielten. Die Waschschritte wurden dreifach mit PBS-Puffer ohne Ca²⁺ und Mg²⁺ (erhältlich von Biochrom) durchgeführt. Die Präparate wurden für 30 Minuten in gepufferter Lösung (pH 8,8) angefärbt mit Neufuchsin als Haftungsagens und einem Naphtholsalz als Kupplungsagens (beides von Sigma), gegengefärbt mit Mayer's Haemalum (Merck, Darmstadt, Deutschland), abgedeckt und unter einem Lichtmikroskop beurteilt.

### Isolierung und Quantifizierung von Proteinen

Um zelluläre Proteine zu isolieren, wurden Zellkulturen zweimal mit PBS gewaschen, direkt in den Kulturgefäßen mittels einer kalten Lösung lysiert, die aus 50 mM HEPES, 1% Nonidet P-40 (erhältlich von ICN, Aurora, OH, USA), 150 mM NaCl sowie einem Proteaseinhibitor (Complete™ Mini; erhältlich von Boehringer Mannheim) bestand, anschließend abgeschabt und in kleinen Zentrifugationsgefäßen geerntet. Das gewonnene Material wurde mittels Ultraschalleinwirkung homogenisiert, zentrifugiert, und die Überstände wurden auf Eis gehalten. Bicinchoninsäure (BCA-Protein-Assay; erhältlich von Pierce, Rochford, IL, USA) wurde zugegeben, um das Gesamtprotein sichtbar zu machen, und die Proteinkonzentration wurde durch Messung der Absorption bei 550 nm quantifiziert.

### Westernblot-Analyse

Aliquots des isolierten Proteins (20 µg) wurden für 15 Minuten auf 95°C erhitzt. Eindimensionale SDS/PAGE-Elektrophorese wurde bezüglich jeder Probe auf 7,5-prozentigen Gelen durchgeführt. Dann wurden Proteine auf eine Transfermembran (Immobilon-P aus PVDF; erhältlich von Millipore, Eschborn, Deutschland) unter Verwendung eines Standard-Blotsystems (erhältlich von Bio-Rad, München, Deutschland) übertragen. Die Blots wurden mit primärem, monoklonalem Antikörper für 60 Minuten bei Raumtemperatur inkubiert und dann mit Meerrettichperoxydase-konjugiertem Ziege/Anti-Maus-monoklonalem Antikörper bzw. Ziege/Anti-Kaninchen-monoklonalem Antikörper (erhältlich von Oncogene Science) in einer Verdünnung von 0,2 µg/ml für 60 Minuten bei Raumtemperatur inkubiert. Nach gründlichem Waschen wurden Signale durch die Chemilumineszenzmethode unter Verwendung einer Standard-Assay (ECL, erhältlich von Amersham, Braunschweig) auf Röntgenstrahl-empfindlichen Filmen (XAR 5; erhältlich von Kodak, Rochester, NY, USA) sichtbar gemacht, wobei mehrere Bestrahlungsintervalle eingestellt wurden.

### Ölrot- und Nilrot-Anfärbung

In Kammerobjektträgern wachsende Zellen wurden entweder mit 0,6 %-iger Lösung von Ölrot (Sigma) in 60 %.igem Isopropanol für 15-120 min. oder mit 1 µg/ml Nilrot-Farbstoff (erhältlich von Kodak) für 15 min. bei Raumtemperatur inkubiert, wie von Xia et al. (1989) (*supra*) beschrieben. Die Kulturen wurden dann unter einem Lichtmikroskop (nach Ölrotanfärbung) oder einem Fluoreszenzmikroskop unter Verwendung eines 450-500 nm Bandpass-Anregungsfilters mittels Lichtemission über 528 nm (nach Nilrotanfärbung) beobachtet.

### Durchflußzytometrie

Dispergierte, nicht-markierte Zellen wurden zur Bestimmung der zellgröße mittels eines herkömmlichen Sorters sortiert, während mit Nilrot-Farbstoff (erhältlich von Kodak) markierte Zellen zur Bestimmung des Lipidgehalts durchflußzytometrisch auf Fluoreszenzbasis bestimmt wurden. Es wurden 10.000 Zellen pro Probe untersucht.

### Markierung und Extraktion von Lipiden

Zellkulturen wurden in Kulturmedium für zwei Tage gehalten und dann mit einem radioaktiven Puls versetzt über das Natriumsalz von [2-¹⁴C]-Essigsäure (45-60 mCi/mmol; erhältlich von Dupond-NEN, Boston, MA, USA) in einer Konzentration von 0,5 µCi/ml in RPMI-1640-Medium, zu dem 2mM L-Glutamin, 10 % hitzeinaktiviertes FCS, und 100 IU/ml Penicillin und 100 µg/ml Streptomycin zugesetzt war. Danach wurde für weitere 24 Stunden inkubiert.

Lipide wurden aus kultivierten Zellen und aus dem überstehenden Kulturmedium isoliert und aufgetrennt nach neutralen Lipiden, Fettsäuren und Phospholipiden (siehe Seifert et al. J. Invest. Dermatol. 1997;108:375).

Die Größentrennung nach Fraktionen und die Sichtbarmachung von neutralen Lipiden und freien Fettsäuren wurden durch Hochleistungs-Dünnschichtchromographie (HPTLC) erhalten, die auf 20x10 cm² großen Silicagel-beschichteten Glasplatten (erhältlich von Merck, Darmstadt, Deutschland) durchgeführt wurden. Die Platten wurden mit n-Hexan vorbehandelt und für 24 Stunden getrocknet. Die Proben wurden durch einen automatischen Lipid-Applikator (Linomat IV; Camag, Berlin, Deutschland) aufgetragen. Chromatogramme der neutralen Lipide wurden in einer n-Hexan/Diethylether-Lösung (9:1) auf 9 cm entwickelt, getrocknet und in einer Lösung von Chloroform/Diethylether/Ethylessigsäure (80:4:16) auf 4,5 cm nachentwickelt. Zur Belichtung wurden Belichtungsbögen (TR2040S, erhältlich von Fuji, Tokyo, Japan) eingesetzt, welche dann unter Verwendung eines Bildanalysiergerätes ("BAS 1000 Bio-Imaging Analyser", Fuji) abgescannt wurden. Lipidstandards wurden als Vergleichsproben eingesetzt.

### Untersuchung des Wachstumsverhaltens

Die Zellen wurden in 96-Well-Kulturplatten bei Dichten von 0,5 bis 4x10³ Zellen/Well angesät. Die Zellproliferation wurde durch den 4-Methylumbelliferyl-Heptanoat-Fluoreszenzassay beurteilt und automatisch gemessen (Zouboulis et al. Melanoma. Res. 1991;1:91-95).

Hierfür wurde am Tag der Beurteilung das Kulturmedium entfernt, die Zellen wurden zweimal mit PBS gewaschen, und 100 µl einer 100 µg/ml-Lösung von 4-Methylumbelliferyl-Heptanoat (Sigma, Deisenhofen, Deutschland) in PBS wurden zu jedem Well hinzugegeben. Die Platten wurden dann für 30 Minuten bei 37°C inkubiert, und die abgegebene Fluoreszenz wurde mit einem geeigneten Fluoreszenz-Meßgerät gemessen (Titertec Fluoroscan II; Flow, Meckenheim, Deutschland). Fluoreszenz-Einheiten wurden erhalten bei 355 nm Anregungs- und 460 nm Emissions-Filtern.

### Behandlung mit 5α-Dihydrotestosteron und Retinoiden

5α-Dihydrotestosteron (5α-DHT; Sigma) wurden in DMSO aufgelöst und anschließend in serum- und phenolfreiem modifiziertem DMEM/Ham's F12-Medium (1:1) (Gibco-BRL) mit 2 Mm n-Acetyl-L-Alanyl-L-Glutamin eingebracht, welches mit 5 ng/ml EGF, 50 µg/ml Rinderhypophysenextrakt, 1 mg/ml Fettsäure-freiem Rinderserumalbumin (Böhringer Mannheim) und 50 µg/ml Gentamycin versetzt war, so daß sich eine Endkonzentration von 10⁻⁶ M 5α-DHT und 0,1% DMSO ergab. 0,1% DMSO allein diente als Kontrolle. Die Zellen (0,5 bis 2 x 10³ pro Well) wurden für 18 Tage mit 5α-DHT behandelt.

Zur Behandlung mit Retinoiden wurden all-*trans*-Retinsäure, 13-cis-Retinsäure und Acitretin in DMSO gelöst und anschließend in serumfreies modifiziertes DME-Medium/Ham's F12-Medium (1:1) mit 2 nM N-Acetyl-L-Alanyl-L-Glutamin eingebracht, welches mit 5 ng/ml EGF, 50 µg/ml Rinderhypophysenextrakt, 1 mg/ml fettsäurefreiem Rinderserumalbumin und 50 µg/ml Gentamycin versetzt war, so daß sich eine Endkonzentration von 10⁻⁷ M Retinoid und 0,1% DMSO ergab. 0,1 % DMSO allein diente als Kontrolle. Retinoide wurden unter abgedunkeltem gelbem Licht gehandhabt. Die Zellen (0,5 bis 1 x 10³ pro Well) wurden für neun Tage mit den Retinoiden behandelt.

### Statistische Analyse

Wachstumsstudien wurden unter Zugrundelegung von sechsfachen Ansätzen in 96-Well-Platten beurteilt. Alle anderen Untersuchungen wurden in Triplikat-Ansätzen durchgeführt.

### Beispiel 1

### Transfektion normaler menschlicher Sebozyten

Der zur Transfektion normaler menschlicher Sebozyten verwendete Vektor mit der Bezeichnung pSVT war ein Plasmidkonstrukt auf der Basis von PBR 322, welcher Sequenzen für das Große-T-Protein von SV-40 aufwies, wobei die Proteinexpression durch das Long-Terminal-Repeat des Rous Sarcoma Virus angetrieben wurde (siehe Dutt et al. Oncogene. 1990;5:195-200; Wang et al. In. Vitro. Cell. Dev. Biol. 1991;27A:63-74). Die zweite Subkultur normaler menschlicher Sebozyten wurde auf 50% Konfluenz in 35 mm-Kulturgefäßen (Becton Dickinson, Plymouth, UK) wachsen gelassen and zur Transfektion eingesetzt. Die Transfektion wurde auf der Basis einer Gentransfermethode mittels Anwendung kationischer Lipide durchgeführt. Dazu wurde das LIPOFECTIN-Reagens eingesetzt, das eine 1:1 (w/w) liposomale Formulierung der kationischen Lipide DOTMA (1,2-Diolyloxypropyl-3-trimethylammoniumchlorid) und DOPE (Diolylphosphatidylethanolamin) in membrangefiltertem Wasser (1 mg/ml) beinhaltet. Hierzu wurde das Kulturmedium entfernt, die Kulturzellen wurden zweimal mit serumfreiem Medium (Opti-MEM von Gibco-BRL) gewaschen und in diesem Medium für vier Stunden inkubiert. Das Medium wurde dann durch eine Transfektionsmischung ersetzt, die aus einer Antibiotika-freien Menge Opti-MEM-Medium (1,5 ml) mit einer geeigneten Menge des LIPOFECTIN-Reagenses (Gibco-BRL; 5-30 µl, am besten 1,5 Vol.-%) sowie einer geeigneten Menge an pSVT-DNA (1-10 µg) in einer Lösung mit 0,5 ml PBS (DNA-End-Konzentration am besten 0,5 Gew.-%) bestand. Die Kulturen wurden für 24 h bei 37°C in feuchter Atmosphäre inkubiert, die 5% CO₂ enthielt. Die Kulturen wurden schließlich zweimal mit Kulturmedium gewaschen und, wie oben beschrieben, weiter im Sebozyten-Kulturmedium gehalten.

Nach der Transfektionsprozedur wurde eine dramatisch verringerte Lebensfähigkeit der pSVT-behandelten Sebozyten im Laufe von vier Wochen beobachtet. Es folgte aber insbesondere beim Einsatz optimaler Mengen an LIPOFECTIN-Reagens und pSVT-DNA das Auftauchen proliferierender Sebozytenkolonien. Diese Zellen (SZ95) konnten bis heute über 50mal passagiert werden. Sie sind nach wie vor lebensfähig über die Beobachtungszeit von 4½ Jahren.

### Beispiel 2

### Klonierung der immortalisierten menschlichen Sebozyten

Die so immortalisierten menschlichen Sebozyten SZ95 wurden in 96-Well-Kulturplatten ausgesät in einer Verdünnungsreihe mit geometrisch absteigenden Zellzahlen von 1 x 10² Zellen in der ersten Reihe bis zum Erreichen von theoretisch null Zellen in der letzten Reihe (Zouboulis et al. in "Das maligne Melanom der Haut", C.E. Orfanos und C. Garbe (Hrsg.) Zuckschwerdt, München, Deutschland: 1990;158-168). Die Zellen wurden in Standardkulturmedium gehalten, welches mit 5 ng/ml EGF und 3 ng/ml KGF versetzt war. Wachsende Zellen wurden als Klone betrachtet, wenn sie von einer einzelnen Zelle pro Well stammten, was durch lichtmikroskopische Untersuchungen beobachtet werden konnte. Somit wurden klonierte SZ95-Zellen erhalten.

### Beispiel 3

### Charakterisierung der immortalisierten menschlichen Sebozyten

### Nachweis des SV-40-Großen-T-Antigens

Die Expression des Großen-T-Antigens von SV-40 in immortalisierten Sebozyten wurde immunzytochemisch und mittels Westernblot-Analyse unter Verwendung eines monoklonalen antihumanen SV-40-Groß-T-Ag-Antikörpers aus Mausserum (Oncogene Science, Cambridge, MA, USA), der für die immunzytochemische Analyse auf 1:1000 und für die Westernblot-Analyse 1:100 verdünnt war, nachgewiesen. Menschliche normale epidermale Keratinozyten, dermale Fibroblasten und - als Positivkontrolle - durch SV-40-Groß-T-Antigen immortalisierte endotheliale Zellen HMEC-1 (siehe WO-A-92/17569) wurden zum Vergleich eingesetzt.

Die immunozytochemische Untersuchung der nach Beispiel 1 immortalisierten Sebozyten mit dem monoklonalen Antikörper gegen das Groß-T-Antigen von SV-40 ergab eine starke, überwiegend nukleäre, teils auch zytoplasmatische Anfärbung (siehe Fig. 2a). Normale Keratinozyten und Fibroblasten waren gleichmäßig negativ gegenüber dem SV-40-Groß-T-Protein, und die HMEC-1-Zellen als Positivkontrolle zeigten überwiegend eine nukleäre, teils auch zytoplasmatische, Färbung für das SV-40-Groß-T-Protein (siehe Fig. 2b).

Fig. 2c zeigt die Ergebnisse der Westernblot-Analyse der SV-40-Groß-T-Antigenexpression in nicht-tranfizierten normalen menschlichen Sebozyten (Spur 1), in normalen menschlichen epidermalen Keratinozyten (Spur 2), in immortalisierten Sebozyten gemäß der Erfindung (34. Subkultur; Spur 3), sowie in verschiedenen klonierten Sebozyten gemäß der Erfindung (Spuren 4, 5 und 6). Eine Bande bei 94 kD wurde für die immortalisierte Sebozytenlinie sowie ihrer Klone nachgewiesen, was die Expression des SV-40-Großen-T-Proteins bestätigte (siehe Harlow et al. J. Virol. 1981;39:861-869).

### Phänotypische Charakterisierung der erfindungsgemäßen, immortalisierten Sebozyten

Die Morphologie der gemäß Beispiel 1 immortalisierten Sebozyten SZ95 war epithelial und zeichnete sich durch eine polymorphe Erscheinung mit Zellen unterschiedlicher Größe aus, wobei zahlreiche Tröpfchen im Zytoplasma beobachtet werden konnten (siehe Fig. 1b und c).

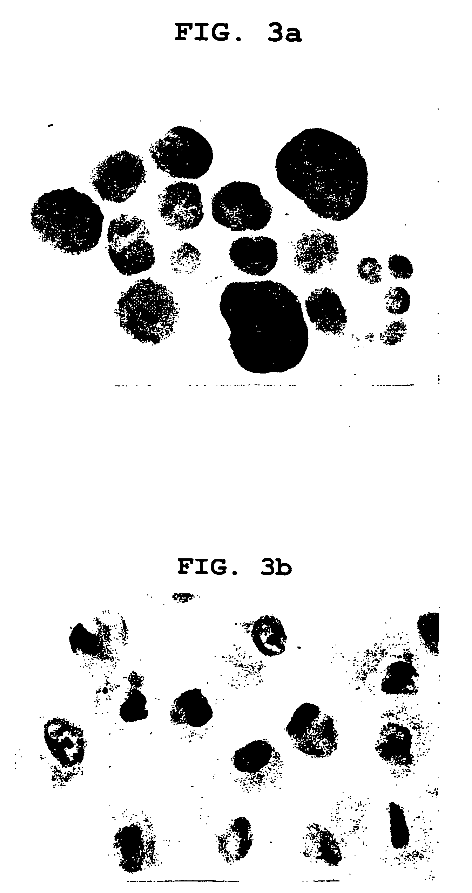

Immunozytochemische Untersuchungen mit entsprechenden Antikörpern ergaben für die erfindungsgemäßen, immortalisierten Sebozyten ein positives Resultat gegen das Talgdrüsenantigen - im Gegensatz zu normalen epidermalen Keratinozyten, die nicht mittels des monoklonalen Antikörpers gegen das Talgdrüsenantigen angefärbt wurden (siehe Fig. 3). Die Fig. 3. zeigt die immunzytochemischen Ergebnisse auf Zytozentrifugationspräparate (a) der erfindungsgemäßen, immortalisierten Sebozyten sowie (b) normaler menschlicher epidermaler Keratinozyten. Die Präparate waren mit einem monoklonalen Antikörper gegen das Talgdrüsenantigen angefärbt. Während die erfindungsgemäßen, immortalisierten Sebozyten eine positive zytoplasmatische Färbung aufwiesen, waren die normalen menschlichen epidermalen Keratinozyten nicht gefärbt.

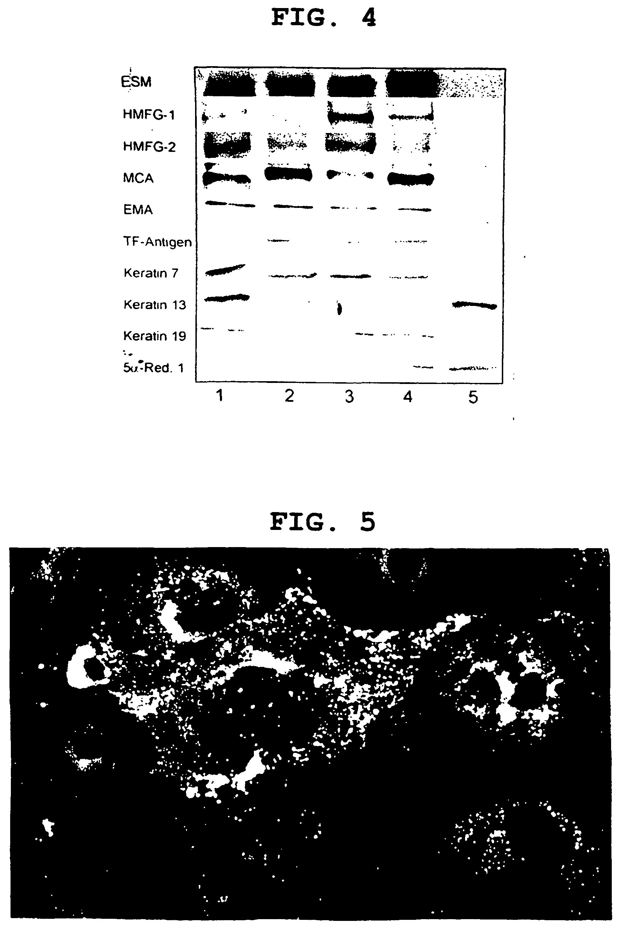

Darüber hinaus wurde über die Westernblot-Analyse die Expression der Keratine 7, 13 und 19 sowie mehrerer Proteine der humanen polymorphen epithelialen Muzingruppe in den immortalisierten Sebozyten nachgewiesen, wohingegen menschliche Keratinozyten lediglich das Keratin 13 exprimierten (siehe Fig. 4). Für die Westernblot-Analyse gemäß Fig. 4 wurden das extrahierte Gesamtprotein immortalisierter Sebozyten gemäß der Erfindung (34. Subkultur; Spur 1), verschiedener klonierter immortalisierter Sebozyten gemäß der Erfindung (Spuren 2, 3 und 4) sowie normaler menschlicher epidermaler Keratinozyteh (Spur 5) aufgetragen, und zwar zur Identifizierung der Expression von humanem epithelialem Sialomucin (ESM) (>400 kD), humanem Milchfett-Globulin-1 (HMFG-1) (400 kD), humanem Milchfett-Globulin-2 (HMFG-2) (80-400 kD), Muzin-ähnlichem, Karzinomassoziiertem Antigen (MCA) (350 kD), epithelialem Membranantigen (EMA) (250-400 kD), Thomsen-Friedenreich-Antigen (TF-Antigen) (155 kD), Keratin 7 (54 kD), Keratin 13 (54 kd), Keratin 19 (40 kD) sowie der 5α-Reduktase vom Typ 1 (5α-Red. 1)(21-27 kD). Die immortalisierte Zellinie sowie ihre Klone gemäß der Erfindung exprimierten alle untersuchten Proteine, wohingegen Keratinozyten nur das Keratin 13 und die 5α-Reduktase vom Typ 1 exprimierten.

### Lipidsynthese

Das Anfärben mit Nilrot und der Beurteilung mittels Fluoreszenzmikroskopie zeigten die Gegenwart von Lipiden im Zellzytoplasma. In den erfindungsgemäßen, immortalisierten Sebozyten SZ95 (siehe Fig. 5), die mit auf neutrale Lipide gerichtetem Nilrot-Fluoreszenzfarbstoff angefärbt wurden, ergaben einzelne oder in Gruppen vorliegende Lipidtröpfchen, die im Zytoplasma der Sebozyten beliebig verteilt waren. Die erfindungsgemäßen, immortalisierten Sebozyten verringerten ihren Lipidgehalt von 510 Fluoreszenzeinheiten pro Zelle (Mittelwert) in serumhaltigem Medium auf 429 Fluoreszenzeinheiten pro Zelle (Mittelwert; d.h. minus 16%) in serumfreiem Medium, was durch fluoreszenzzytrometrische Untersuchungen von Nilrot-angefärbten Zellen nachgewiesen wurde.

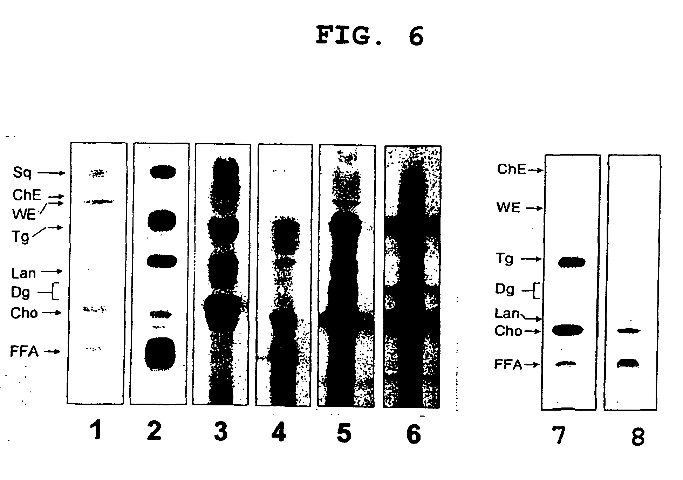

Die erfindungsgemäßen, immortalisierten Sebozyten gemäß Beispiel 1 synthetisierten mehrere Fraktionen neutraler Lipide, darunter die typisch Talgdrüsenlipide Squalen und Wachsester sowie Triglyzeride, Cholesterol, Cholesterolester, Diglyzeride, Lanosterol und freie Fettsäuren. Dies wurde über 25 bis 40 Subkulturen hinweg beobachtet. Dies ist in Fig. 6 gezeigt, wo HPTLC-fraktionierte Lipide nach der Pulsaufnahme von radioaktiv markiertem Natriumacetat über eine radiometrische Bildauswertung detektiert wurden bei zwei ausgewählten, immortalisierten und klonierten Sebozytenkulturen (siehe Spuren 3 und 4 bzw. 5 und 6). Wie die Spuren 3, 4 und 5 zeigen, synthetisierten die Zellen mehrere Fraktionen neutraler Lipide, darunter Squalen (Sq), Wachsester (WE) sowie Triglyzeride (Tg), Cholesterol (Cho), Cholesterolester (ChE), Diglyzeride (Dg), Lanosterol (Lan) sowie freie Fettsäuren (FFA). Alle neutralen Lipide wurden ebenso, wenn auch in geringerem Ausmaß, in den extrazellulären Überständen (siehe Spur 6) gefunden. Zum Vergleich wurden in Spur 1 Lipidstandards, in Spur 2 menschliches Sebum und in Spur 4 freie Fettsäuren, die aus Zellen extrahiert waren, aufgetragen. Als weiteren Vergleich zeigen die Spuren 7 und 8 die Resultate von Keratinozyten, wobei Spur 7 die Anwesenheit von hauptsächlich Cholesterol und Triglyzeriden in den Zellen anzeigt, während aus dem Überstand (Spur 8) überwiegend Cholesterin gefunden wurde.

### Proliferationsstudien

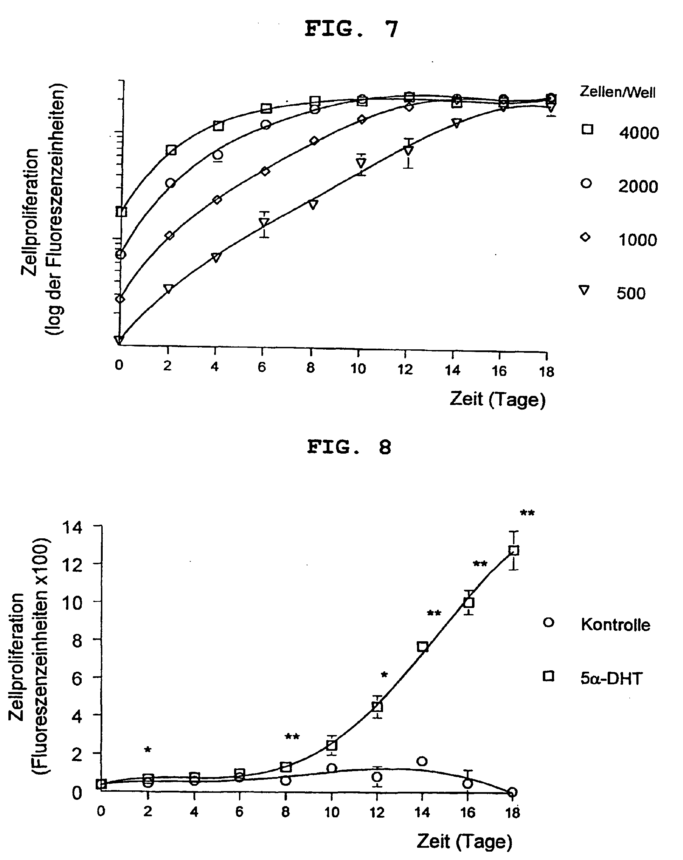

Ein logarithmisches Proliferationsmuster der immortalisierten Sebozyten gemäß der Erfindung wurden unter normalen Kulturbedingungen mit Populations-Verdopplungszeiten von 52,4 +/- 1,6 unabhängig von den ursprünglichen Kulturzelldichten nachgewiesen. Hierzu zeigt Fig. 7 die Proliferation einer immortalisierten, klonierten Sebozytenzellinie (SZ95) über 18 Tage in Sebozytenmedium.

Die Proliferation der immortalisierten Sebozyten war verringert nach Zugabe von serumfreiem Medium, wurde aber wieder aufgehoben nach Zugabe von 5α-DHT. Dies ist in Fig. 8 für einen beispielhaften Sebozytenklon gezeigt, wobei die Proliferation der Zellen (Aussaat 2000 pro Well) über 18 Tage in serumfreiem Medium (Kontrolle), sowie in serumfreiem Medium, welches mit 10⁻⁶ M 5α-DHT versetzt war, beobachtet wurde. Nach dem 8. Tag erhöhte das 5α-DHT die Proliferation der Zellen beträchtlich, was sich an der festgestellten Populations-Verdopplungszeit von 136 Stunden (Kontrolle) und 53,7 Stunden (5α-DHT-behandelte Zellen) zeigte (*, p<0.05; **, p<0.01).

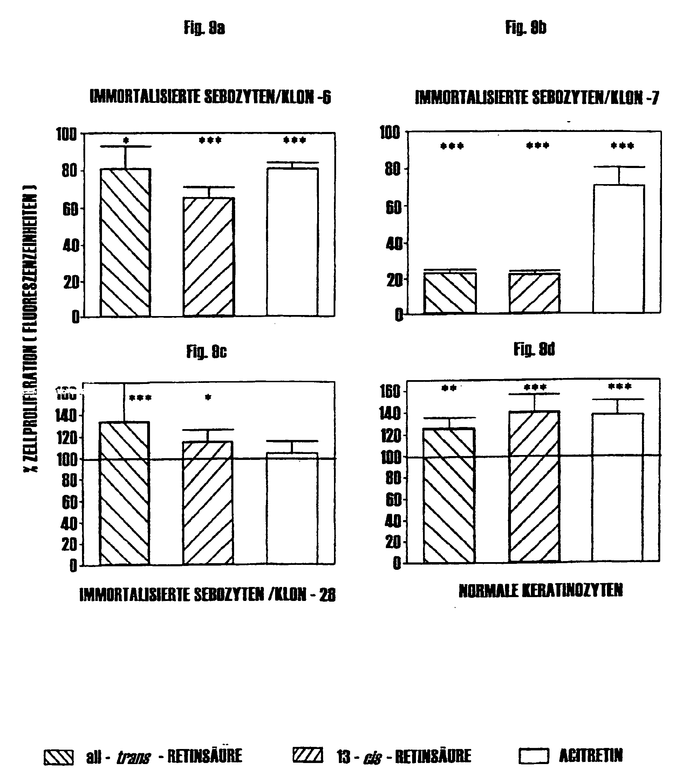

Das Einwirken von Retinoiden auf die immortalisierten Zellen zeigte ein unterschiedliches Ansprechen des Proliferationsverhaltens. Während manche Klone durch Retinoide in der Proliferation inhibiert wurden (typischerweise verschieden stark in der Reihenfolge 13-cis-Retinsäure > all-trans-Retinsäure >> Acitretin), wurden andere Klone in der Proliferation stimuliert (beispielsweise durch all-trans-Retinsäure und 13-*cis*-Retinsäure) entsprechend der Proliferationsantwort von normalen menschlichen epidermalen Keratinozyten. Dies ist in Fig. 9 gezeigt (*, p<0.05; **, p<0.01; ***, p<0.001).

## Patentansprüche

1. Sebozyten, die immortalisiert sind und vom Menschen stammen, erhältlich durch die Transfektion von Sebozyten mit einer DNA, die DNA-Sequenzen umfasst, welche für das Große-T-Protein von SV-40 kodieren, **dadurch gekennzeichnet, dass** sie Merkmale von normalen, nicht-transfizierten und differenzierenden Sebozyten aufweisen.

2. Sebozyten nach Anspruch 1, **dadurch gekennzeichnet, dass** sie von der menschlichen Talgdrüse stammen.

3. Sebozyten nach Anspruch 2, **dadurch gekennzeichnet, dass** die Talgdrüsenzellen Gesichtstalgdrüsenzellen sind.

4. Sebozyten nach einem der vorangehenden Ansprüche, **dadurch gekennzeichnet, dass** sie in Form einer Zelllinie vorliegen.

5. Sebozyten nach einem der vorangehenden Ansprüche, **dadurch gekennzeichnet, dass** ihre Proliferation mittels Androgenen und/oder Retinoiden veränderbar ist.

6. Sebozyten nach einem der vorangehenden Ansprüche, **dadurch gekennzeichnet, dass** sie kloniert sind.

7. Die menschliche Sebozyten-Zelllinie DSM ACC2383.

8. Verwendung der Sebozyten gemäß einem der Ansprüche 1 bis 6 oder der menschlichen Sebozyten-Zellinie gemäß Anspruch 7 zur Herstellung eines Medikaments oder Präparats für diagnostische, für therapeutische oder für kosmetische Ansätze.

9. Verwendung der Sebozyten gemäß einem der Ansprüche 1 bis 6 oder der menschlichen Sebozyten-Zelllinie gemäß Anspruch 7 zur Untersuchung der Physiologie oder der Pathophysiologie der menschlichen oder der tierischen Talgdrüse.

10. Verwendung der Sebozyten gemäß einem der Ansprüche 1 bis 6 oder der menschlichen Sebozyten-Zelllinie gemäß Anspruch 7 zur Untersuchung der Entstehung der Akne und/oder der Seborrhoe und/oder anderer Erkrankungen.

11. Verwendung der Sebozyten gemäß Anspruch 10, wobei die zu untersuchenden anderen Krankheiten Hautkrankheiten sind, bei denen die Talgdrüsenfunktion eine Rolle spielt oder spielen kann.

12. Verwendung der Sebozyten gemäß einem der Ansprüche 1 bis 6 oder der menschlichen Sebozyten-Zelllinie gemäß Anspruch 7 zum Testen von Anti-Akne- und/oder Anti-Seborrhoe-Verbindungen oder -Mitteln.

13. Verwendung der Sebozyten gemäß einem der Ansprüche 1 bis 6 oder der menschlichen Sebozyten-Zelllinie gemäß Anspruch 7 zum Testen von Verbindungen oder Mitteln gegen Erkrankungen.

14. Verwendung der Sebozyten gemäß Anspruch 13, wobei die Erkrankungen Hautkrankheiten sind, bei denen die Talgdrüsenfunktion eine Rolle spielt oder spielen kann.

15. Verwendung der Sebozyten gemäß einem der Ansprüche 1 bis 6 oder der menschlichen Sebozyten-Zelllinie gemäß Anspruch 7 zur Entwicklung von einfachen oder komplexen Zellkultursystemen.

16. Verwendung der Sebozyten gemäß einem der Ansprüche 1 bis 6 oder der menschlichen Sebozyten-Zelllinie gemäß Anspruch 7 zur Bildung von oder zum Einsatz in dreidimensionalen Zellansammlungen oder Konstruktionen organähnlicher Strukturen.

17. Verwendung der Sebozyten gemäß einem der Ansprüche 1 bis 6 oder der menschlichen Sebozyten-Zelllinie gemäß Anspruch 7 zur Herstellung von Produkten, die von diesen Zellen stammen.

18. Verwendung gemäß Anspruch 17, wobei die Zellprodukte Lipide, Plasmide, Vektoren, Proteine, die von den Zellen exprimiert werden, und/oder DNA- oder RNA-Sequenzen dieser Proteine sind.

## Claims

1. Sebocytes which are immortalized and of human origin, obtainable by transfection of sebocytes with a DNA comprising DNA sequences coding for the large T protein of SV-40, **characterized in that** they possess features of normal, non-transfected and differentiating sebocytes.

2. Sebocytes according to Claim 1, **characterized in that** they originate from the human sebaceous gland.

3. Sebocytes according to Claim 2, **characterized in that** the sebaceous gland cells are facial sebaceous gland cells.

4. Sebocytes according to any one of the preceding daims, **characterized in that** they exist in the form of a cell line.

5. Sebocytes according to any one of the preceding claims, **characterized in that** their proliferation is modifiable by means of androgens and/or retinoids.

6. Sebocytes according to any one of the preceding claims, **characterized in that** they are cloned.

7. The human sebocyte cell line DSM ACC2383.

8. Use of sebocytes according to any one of Claims 1 to 6 or of the human sebocyte cell line according to Claim 7 for the production of a medicament or preparation for diagnostic, therapeutic or cosmetic purposes.

9. Use of sebocytes according to any one of Claims 1 to 6 or of the human sebocyte cell line according to Claim 7 for investigation into the physiology or pathophysiology of the human or animal sebaceous gland.

10. Use of sebocytes according to any one of Claims 1 to 6 or of the human sebocyte cell line according to Claim 7 for investigation into the origins of acne and/or seborrhoea and/or other diseases.

11. Use of sebocytes in accordance with Claim 10 where the other diseases under investigation are skin diseases in which the function of the sebaceous gland is or may be a contributory factor.

12. Use of sebocytes according to any one of Claims 1 to 6 or of the human sebocyte cell line according to Claim 7 for the testing of anti-acne and/or anti-seborrhoea compounds or agents.

13. Use of sebocytes according to any one of Claims 1 to 6 or of the human sebocyte cell line according to Claim 7 for the testing of compounds or agents for combating diseases.

14. Use of sebocytes in accordance with Claim 13 where the diseases are skin diseases in which the function of the sebaceous gland is or may be a contributory factor.

15. Use of sebocytes according to any one of Claims 1 to 6 or of the human sebocyte cell line according to Claim 7 for the development of simple or complex cell culture systems.

16. Use of sebocytes according to any one of Claims 1 to 6 or of the human sebocyte cell line according to Claim 7 for the formation of, or for use in, three-dimensional cell-accumulations or constructions of organ-like structures.

17. Use of sebocytes according to any one of Claims 1 to 6 or of the human sebocyte cell line according to Claim 7 for the production of products derived from these cells.

18. Use according to Claim 17 where the cell products are lipids, plasmids, vectors, proteins expressed by the cells, and/or DNA or RNA sequences of these proteins.

## Revendications

1. Sébocytes qui sont immortalisés et d'origine humaine, pouvant être obtenus par transfection de sébocytes avec un ADN comprenant des séquences d'ADN qui codent pour la protéine grand T du SV-40, **caractérisés en ce qu'**ils présentent les caractéristiques de sébocytes normaux non transfectés et se différenciant.

2. Sébocytes selon la revendication 1, **caractérisés en ce qu'**ils proviennent des glandes sébacées humaines.

3. Sébocytes selon la revendication 2, **caractérisés en ce que** les cellules de glandes sébacées sont des cellules de glandes sébacées du visage.

4. Sébocytes selon l'une des revendications précédentes, **caractérisés en ce qu'**ils se présentent sous la forme d'une lignée cellulaire.

5. Sébocytes selon l'une des revendications précédentes, **caractérisés en ce que** leur prolifération peut être modifiée au moyen d'androgènes et/ou de rétinoïdes.

6. Sébocytes selon l'une des revendications précédentes, **caractérisés en ce qu'**ils sont clonés.

7. La lignée cellulaire de sébocytes humains DSM ACC2383.

8. Utilisation des sébocytes selon l'une des revendications 1 à 6 ou de la lignée cellulaire de sébocytes humains selon la revendication 7 pour produire un médicament ou une préparation destinée à des applications diagnostiques, thérapeutiques ou cosmétiques.

9. Utilisation des sébocytes selon l'une des revendications 1 à 6 ou de la lignée cellulaire de sébocytes humains selon la revendication 7 pour étudier la physiologie ou la physiopathologie des glandes sébacées humaines ou animales.

10. Utilisation des sébocytes selon l'une des revendications 1 à 6 ou de la lignée cellulaire de sébocytes humains selon la revendication 7 pour étudier la genèse de l'acné et/ou de la séborrhée et/ou d'autres affections.

11. Utilisation des sébocytes selon la revendication 10, dans laquelle les autres maladies à étudier sont des maladies cutanées dans lesquelles la fonction des glandes sébacées joue ou peut jouer un rôle.

12. Utilisation des sébocytes selon l'une des revendications 1 à 6 ou de la lignée cellulaire de sébocytes humains selon la revendication 7 pour tester des composés ou agents anti-acné et/ ou anti-séborrhéiques.

13. Utilisation des sébocytes selon l'une des revendications 1 à 6 ou de la lignée cellulaire de sébocytes humains selon la revendication 7 pour tester des composés ou agents de lutte contre des affections.

14. Utilisation des sébocytes selon la revendication 13, dans laquelle les affections sont des maladies cutanées dans lesquelles la fonction des glandes sébacées joue ou peut jouer un rôle.

15. Utilisation des sébocytes selon l'une des revendications 1 à 6 ou de la lignée cellulaire de sébocytes humains selon la revendication 7 pour développer des systèmes de culture cellulaire simples ou complexes.

16. Utilisation des sébocytes selon l'une des revendications 1 à 6 ou de la lignée cellulaire de sébocytes humains selon la revendication 7 pour former, ou pour être utilisés dans, des édifices cellulaires en trois dimensions ou dans la construction de structures analogues à des organes.

17. Utilisation des sébocytes selon l'une des revendications 1 à 6 ou de la lignée cellulaire de sébocytes humains selon la revendication 7 pour préparer des produits dérivés de ces cellules.

18. Utilisation selon la revendication 17, dans laquelle les produits cellulaires sont des lipides, des plasmides, des vecteurs, des protéines exprimées par les cellules et/ou des séquences d'ADN ou ARN de ces protéines.
